# EUROPEAN PATENT SPECIFICATION

(11) **EP 2 042 153 B1**
(45) Date of publication and mention of the grant of the patent: **26.11.2014**
(21) Application number: 07739599.4
(22) Date of filing: 26.03.2007
(51) Int. Cl.: A61K 8/37, A61Q 19/02

(54) **SKIN COSMETIC**
HAUTKOSMETIKUM
COSMETIQUE POUR LA PEAU

(30) Priority: 30.03.2006 JP 2006093872
(43) Date of publication of application: 01.04.2009
(73) Proprietor: Kao Corporation, Chuo-ku Tokyo 103-8210 (JP)
(72) Inventor: SUGITA, Jun c/o Basic Research laboratory Kanebo Cosmetics Inc., Kanagawa 250-0002 (JP); NAKAHARA, Michio c/o Basic Research laboratory Kanebo Cosmetics Inc., Kanagawa 250-0002 (JP); IKEMOTO, Takeshi c/o Basic Research laboratory Kanebo Cosmetics Inc., Kanagawa 250-0002 (JP)
(74) Representative: Green, Mark Charles
(86) International application number: PCT/JP2007/056161
(87) International publication number: WO 2007/114095

(56) References cited:
- JP-A- 4 082 814
- JP-A- 7 025 743
- JP-A- 07 025 743
- JP-A- 10 194 961
- JP-A- 10 194 961
- JP-A- 2000 281 539
- JP-A- 2000 281 539
- JP-A- 2001 151 660
- JP-A- 2001 151 660
- JP-A- 2002 241 254
- JP-A- 2002 241 254
- JP-A- 2004 182 600
- JP-A- 2004 182 600
- TAKEYA T ET AL: "Biphenyls, a new class of compound that inhibits platelet-activating factors", CHEMICAL AND PHARMACEUTICAL BULLETIN, PHARMACEUTICAL SOCIETY OF JAPAN, JP, vol. 2, no. 38, 1 February 1990 (1990-02-01), pages 559-561, XP002079398, ISSN: 0009-2363

## Description

### Technical Field

The present invention relates to a skin cosmetic product having an excellent whitening effect to inhibit skin tanning by ultraviolet rays and having high safety.

### Background Art

When skin is exposed to ultraviolet rays, there are various influences in skin. That is, active oxygens, lipid peroxides or the like generated in the skin by ultraviolet exposure causes inflammation and great damage to the skin tissue. The damage causes a loss of skin moisture or gloss, skin texture or the like. Further, when it influences dermis, wrinkles or the like are formed to cause photoaging. Active oxygens generated by ultraviolet rays and various factors released from skin cells by its influence enhance tyrosinase activity in melanocytes. Melanin involved in skin color is produced through oxidation of tyrosine by tyrosinase in melanocytes. Therefore, it is assumed that when tyrosinase is activated by ultraviolet rays, melanin is excessively produced and transferred to epidermal cells, resulting in a change in skin color or skin tanning.

Accordingly, it is essential to inhibit melanogenesis in order to exhibit a whitening effect. Conventionally, whitening cosmetic products including kojic acid, arbutin, hydroquinone monobenzyl ether, hydrogen peroxide or the like have been proposed to prevent skin tanning, spots and freckles and maintain the original light skin. Further cosmetic products including vitamin C or the like have been proposed to inhibit inflammation by ultraviolet rays.

The cosmetic products including arbutin, kojic acid, hydroquinone monobenzyl ether or the like have an effect of lightening the dark skin slightly; however, the effect has not achieved a desired level thereof. Such products do not have an effect of inhibiting inflammation by ultraviolet rays and may have a problem in skin safety. The cosmetic products including vitamin C or the like have a whitening effect and an anti-inflammatory effect; however, there is room to be improved in terms of the degree and stability of the effects. As described above, it is extremely difficult to obtain a whitening cosmetic product having excellent inflammation inhibitory and whitening effects and having high skin safety and sufficient storage stability.

On the other hand, magnolol, honokiol or the like having a biphenol structure is known to inhibit tyrosinase activity (Patent Document 1). However, such biphenol compounds have an unsaturated bond as a partial structure and thus have a problem in safety and stability. The present inventors have already found that a biphenol compound represented by the following general formula (2) has a melanogenesis inhibitory effect (Patent Document 2, Non-Patent Document 1). However, the biphenol compound has poor solubility and high crystallizabilty, and thus there is a limitation of the range of pharmaceutical use in including it for a pharmaceutical product, a quasi drug or a cosmetic product. In addition, the biphenol compound has high polarity and thus has a problem such as poor permeability into the skin. 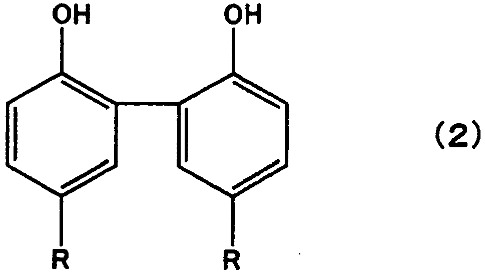 Wherein, R is a hydrogen atom or a linear or branched saturated hydrocarbon group having 1 to 8 carbon atoms.

Patent Document 1: Japanese Unexamined Patent Publication No. Hei 4-82814
Patent Document 2: Japanese Patent No. 2719300
Non-Patent Document 1: "Development of whitening cosmetics with Magnolignan on inhibitory effect of the maturation of tyrosinase", Fragrance Journal, Vol. 34, No. 2, pp. 80-83, 2006

### Disclosure of the Invention

### Problems to be Solved by the Invention

Under such circumstances, an objective of the present invention is to develop a melanogenesis inhibitor which has excellent safety and stability, is widely used for various preparations, and has a high melanogenesis inhibitory effect, and thereby to provide a skin cosmetic product having an excellent whitening effect to inhibit skin tanning by ultraviolet rays and having high safety.

### Means for Solving Problems

As a result of extensive studies to improve drawbacks in the conventional art in view of such circumstances, the present inventors have found that an acylated biphenol compound obtained by acylating a specific biphenol compound has excellent melanogenesis inhibitory effect and safety and has excellent properties as a melanogenesis inhibitor, and that the acylated biphenol compound is an oily compound and has remarkably improved properties to be included to various preparations. It is known that when a biphenol compound is acylated, such a compound having an acyl group with a long carbon chain is generally an oily compound. However, the acylated biphenol compound of the present application is an extremely rare case where such a compound with a short carbon chain is an oily compound.

As described above, the present inventors have found that an acylated biphenol compound having a specific structure has excellent properties as a melanogenesis inhibitor and have finally provided a skin cosmetic product having an excellent whitening effect and having high stability and safety. That is, the first invention of the present application is a melanogenesis inhibitor comprising as an active ingredient an acylated biphenol compound represented by the following general formula (1): 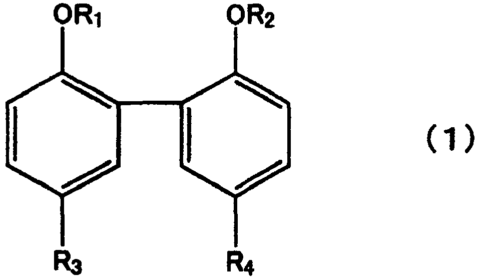 wherein
R₁ and R₂ are each a hydrogen atom or an acyl group having 2 to 10 carbon atoms, and preferably a hydrogen atom or an acyl group having 2 to 8 carbon atoms, provided that both
R₁ and R₂ are not hydrogen atoms; and
R₃ and R₄ are each a hydrogen atom or a linear or branched saturated hydrocarbon group having 1 to 8 carbon atoms, preferably a hydrogen atom or a linear or branched saturated hydrocarbon group having 1 to 5 carbon atoms, and more preferably a propyl group.

The second invention of the present application is a skin cosmetic product including the melanogenesis inhibitor. The above objective of the present invention is achieved by a melanogenesis inhibitor comprising the above acylated biphenol compound having the specific structure as an active ingredient, and a skin cosmetic product including the acylated biphenol compound as an active ingredient.

### Effect of the Invention

The melanogenesis inhibitor of the present invention comprising an acylated biphenol compound having a specific structure as an active ingredient has not only an excellent inhibitory effect for melanin pigment production as originally intended, but also excellent safety without skin irritation or the like. The acylated biphenol compound is an oily compound, has excellent stability and is easily used for various preparations, and is at the same time excellent in transdermal absorption. Therefore, the skin cosmetic product including the acylated biphenol compound exhibits an excellent whitening effect and also has excellent safety, and can be provided in a wide variety of dosage forms.

### Brief Description of the Drawings

Figure 1 is a ¹H-NMR chart of the monoacetyl derivative of Preparation Example 1.
Figure 2 is a ¹H-NMR chart of the diacetyl derivative of Preparation Example 1.
Figure 3 is a ¹H-NMR chart of the dipropionyl derivative of Preparation Example 2.
Figure 4 is a ¹H-NMR chart of the monooctanoyl derivative of Preparation Example 3.
Figure 5 is an organic conceptional diagram created from IOB values of the respective acyl derivatives obtained in Preparation Examples 1 to 3, propylbiphenol used in Comparative Example 1 and representative solvents, where o represents an IO value of propylbiphenol, Δ represents an IO value of the monoacetyl derivative, □ represents an IO value of the diacetyl derivative, × represents an IO value of the dipropionyl derivative, * represents an IO value of the monooctanoyl derivative, and • represents an IO value of the representative solvent shown in Table 2.
Figure 6 is a melanogenesis inhibition curve of the monoacetyl derivative of Preparation Example 1.
Figure 7 is a melanogenesis inhibition curve of the diacetyl derivative of Preparation Example 1.

### Best Mode for Carrying Out the Invention

The following illustrates embodiments of the present inventions in detail. The acylated biphenol compound represented by the general formula (1) which is used in the present invention can be easily synthesized by known chemical synthesis procedures. For example, the compound can be prepared by dimerizing 4-propylphenol, a commercially-available reagent, according to the method of J.C. Pew et al. (Journal of Organic Chemistry, Vol. 28, p. 1048, 1963) and then reacting the dimer with a fatty acid anhydride or fatty acid chloride in the presence of pyridine. Column chromatography or distillation can be used as a method for purifying the compound.

The skin cosmetic product can include a melanogenesis inhibitor comprising the acylated biphenol compound according to the present invention as an active ingredient. The amount of the acylated biphenol compound is preferably 0.001 to 10.0 mass% (hereinafter simply described as "%"), and more preferably 0.01 to 5.0% based on the total amount of the skin cosmetic product. When the amount is less than the lower limit, the intended effect of the present invention is not sufficient, unfavorably. On the other hand, even when the amount is more than the upper limit, there is no improvement of the effect comparable to the increase, unfavorably. In particular, since the acylated biphenol compound used in the present invention has high solubility in a water-soluble organic solvent such as ethanol or a polyhydric alcohol, the skin cosmetic product of the present invention may be an aqueous cosmetic product such as a lotion. In case of an aqueous cosmetic product, the amount of the acylated biphenol compound is preferably 0.01 to 8.0%, and more preferably 0.1 to 5.0% based on the total amount of the cosmetic product. The amount of the water-soluble organic solvent is preferably 1 to 40%, and more preferably 5 to 20% based on the total amount of the cosmetic. Here, the water-soluble organic solvent means solvent compatible with water, specifically including lower alcohols or polyhydric alcohols. The water-soluble organic solvent is preferably one or more lower alcohols selected from ethanol, propanol, isopropanol and butanol and/or one or more polyhydric alcohols selected from ethylene glycol, diethylene glycol, triethylene glycol, polyethylene glycol, propylene glycol, dipropylene glycol, polypropylene glycol, glycerol, diglycerol, polyglycerol, isoprene glycol and 1,3-butylene glycol, and more preferably an organic solvent having an IOB value in the range of 1 to 7, and more preferably in the range of 2 to 5. (The IOB value will be described later.)

The skin cosmetic product of the present invention can include various conventionally known melanogenesis inhibitors, whitening agents, antioxidants or the like, in addition to the melanogenesis inhibitor comprising the acylated biphenol compound as an active ingredient. Also combination with non-acylated biphenol compound corresponding to the acylated biphenol compound of the present application is no problem as long as the effects of the present invention are not impaired.

The skin cosmetic product of the present invention may be used not only as a cosmetic product generally applied to the skin but also as a bath agent. The dosage form may be an aqueous solution, a W/O or O/W emulsion, or granules besides a powder, tablets, or the like using an appropriate excipient. Specific examples of the dosage form include creams, skin milks, lotions, packs, gels, sticks, sheets and cataplasms. When the skin cosmetic product is a skin milk, for example, it can be prepared by a conventional method in which an oily phase and an aqueous phase are dissolved by heating respectively, dispersed by emulsification, and cooled.

The skin cosmetic product of the present invention can be made in the above-described desired dosage form by appropriately including a cosmetic base conventionally used in the cosmetic industry. The amount of the cosmetic base is preferably 90 to 99.999%, and more preferably 95 to 99.99% based on the total amount of the cosmetic product. Examples of the cosmetic base include, in addition to water and the water-soluble organic solvents described above, fats and oils, waxes, hydrocarbons, higher fatty acids, higher alcohols, esters, silicone oils, color pigments such as tar dyes and iron oxide, preservatives such as paraben, anionic surfactants such as fatty acid soap and sodium cetyl sulfate, nonionic surfactants such as polyoxyethylene alkyl ether, polyoxyethylene fatty acid ester, polyoxyethylene polyhydric alcohol fatty acid ester, polyoxyethylene hydrogenated castor oil, polyhydric alcohol fatty acid ester and polyglycerol fatty acid ester, cationic surfactants such as tetraalkylammonium salts, amphoteric surfactants such as betaine surfactants, sulfobetaine surfactants, sulfoamino acid surfactants and sodium N-stearoyl-L-glutamate, natural surfactants such as lecitin and lysophosphatidylcholine, natural polymers such as gelatin, casein, starch, gum arabic, karaya gum, guar gum, locust bean gum, tragacanth gum, quince seed, pectin, carageenan and sodium alginate, semisynthetic polymers such as methylcellulose, hydroxyethylcellulose, hydroxypropylcellulose, sodium carboxymethylcellulose and ethylcellulose, synthetic polymers such as polyvinyl alcohol, polyvinyl methyl ether, polyvinyl alcohol-polyethylene glycol graft copolymers, polyvinylpyrrolidone, sodium polyacrylate, carboxyvinyl polymers and polyethylene oxide polymers, thickeners such as xanthene gum, pigments such as titanium oxide, and antioxidants such as dibutylhydroxytoluene. These can be appropriately included to the extent that the objective of the present invention is not impaired.

### Examples

The present inventions are illustrated in detail by way of the following Examples, Preparation Examples and Comparative Examples. However, the present inventions shall not be limited thereto.

### Preparation Example 1

Anhydrous tetrahydrofuran was added to 5.0 g of 2,2'-dihydroxy-5,5'-di-n-propyl-biphenyl used as a cosmetic raw material (hereinafter abbreviated as "propylbiphenol"), and the mixture was stirred under icecooling. Further, 1.0 ml of pyridine and 6.0 g of acetyl chloride were sequentially added. After four hours, the mixture was worked up according to a conventional method to yield 5.2 g of a crude product.

The resulting product was fractionated by silica gel chromatography [mobile phase: ethyl acetate/n-hexane (1:5)] using Silica Gel 60N (100 to 210 µm) manufactured by Kanto Chemical Co., Inc. to yield 3.0 g of a monoacetyl derivative and 1.9 g of a diacetyl derivative as pale yellow oily compounds, respectively.

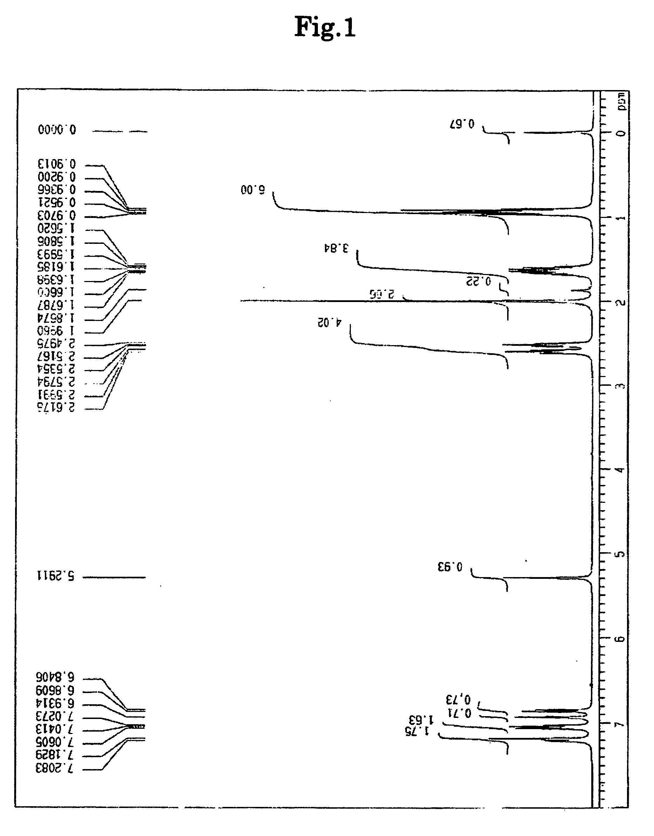

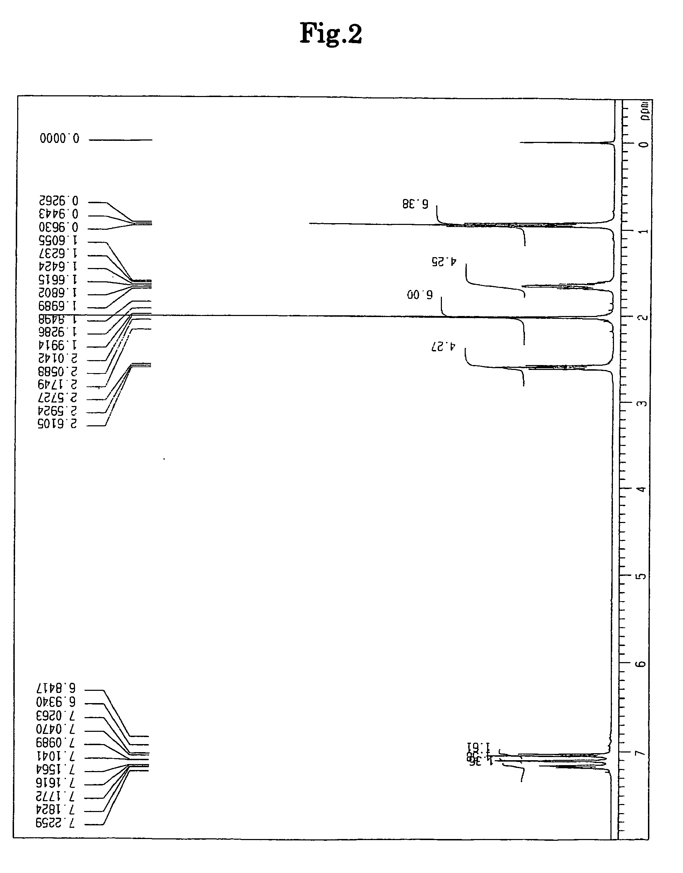

The resulting monoacetyl and diacetyl derivatives of propylbiphenol were identified by ¹H-NMR (400 MHz, CDCl₃) (Figures 1 and 2). The detected NMR signals are shown below.

### (Monoacetyl derivative)

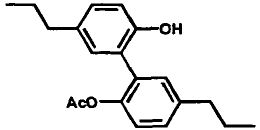

[δ (ppm); 0.94 (m, 6H), 1.63 (m, 4H), 1.99 (s, 3H), 2.51 (t, J = 7.5 Hz, 2H), 2.59 (t, J = 7.5 Hz, 2H), 5.29 (s, 1H), 6.85 (d, J = 8.1 Hz, 1H), 6.93 (s, 1H), 7.02 (s, 1H), 7.05 (d, 7.7 Hz, 1H), 7.19 (m, 2H)]

### (Diacetyl derivative)

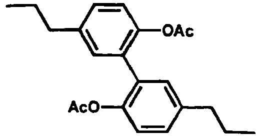

[δ (ppm); 0.94 (t, J = 7.5 Hz, 6H), 1.65 (q, J = 7.5 Hz, 4H), 1.99 (s, 6H), 2.59 (t, J = 7.2 Hz, 4H), 7.03 (d, J = 8.3 Hz, 2H), 7.10 (s, 2H), 7.17 (d, J = 8.3 Hz, 2H)]

### Preparation Example 2

Anhydrous tetrahydrofuran was added to 2.2 g of propylbiphenol and the mixture was stirred under icecooling in the same manner as in Preparation Example 1. Further, 0.5 ml of pyridine and about 3.5 ml of propionyl chloride were sequentially added. After four hours, the mixture was worked up according to a conventional method to yield 2.2 g of a crude product.

The resulting crude product was fractionated by silica gel column chromatography [mobile phase: ethyl acetate/n-hexane (1:4)] using Silica Gel 60N (100 to 210 µm) manufactured by Kanto Chemical Co., Inc. to yield 1.47 g of a dipropionyl derivative as a colorless oily compound.

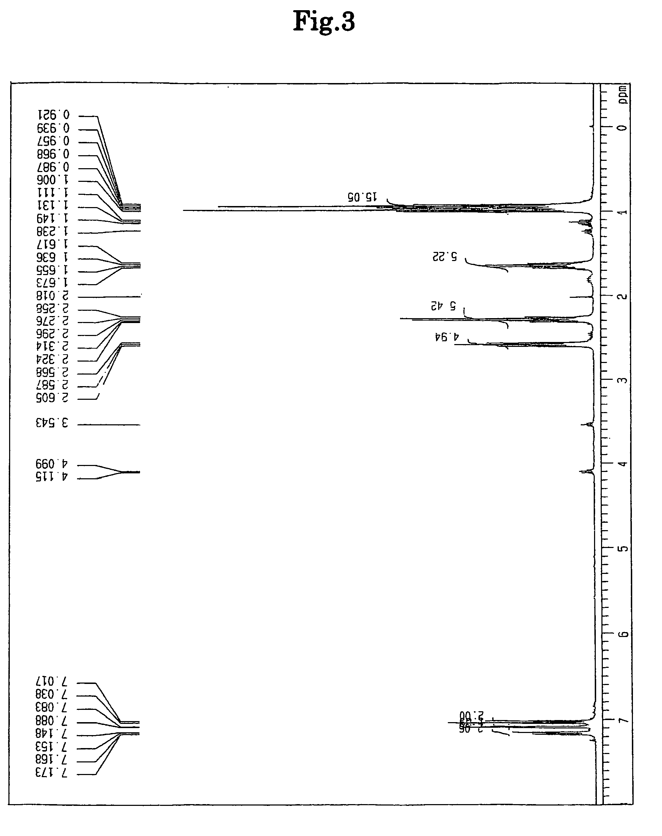

The resulting dipropionyl derivative of propylbiphenol was identified by ¹H-NMR (400 MHz, CDCl₃) (Figure 3). The detected NMR signals are shown below.

### (Dipropionyl derivative)

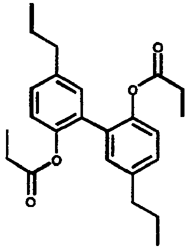

[δ (ppm); 0.93 (t, J = 7.2 Hz, 6H), 0.98 (t, J = 7.6 Hz, 6H), 1.64 (q, J = 7.2 Hz, 4H), 2.28 (q, J = 7.2 Hz, 4H), 2.59 (t, J = 7.2 Hz, 4H), 7.02 (d, J = 8.4 Hz, 2H), 7.08 (s, 2H), 7.16 (dd, J = 2.0, 8.0 Hz, 2H)]

### Preparation Example 3

Anhydrous tetrahydrofuran was added to 1.0 g of propylbiphenol and the mixture was stirred under icecooling in the same manner as in Preparation Example 1. Further, 0.5 ml of pyridine and about 2.5 ml of octanoyl chloride were sequentially added. After four hours, the mixture was worked up according to a conventional method to yield 1.5 g of a crude product.

The resulting crude product was fractionated by silica gel column chromatography [mobile phase: ethyl acetate/n-hexane (1:10)] using Silica Gel 60N (100 to 210 µm) manufactured by Kanto Chemical Co., Inc. to yield 1.25 g of a monooctanoyl derivative as a pale yellow oily compound.

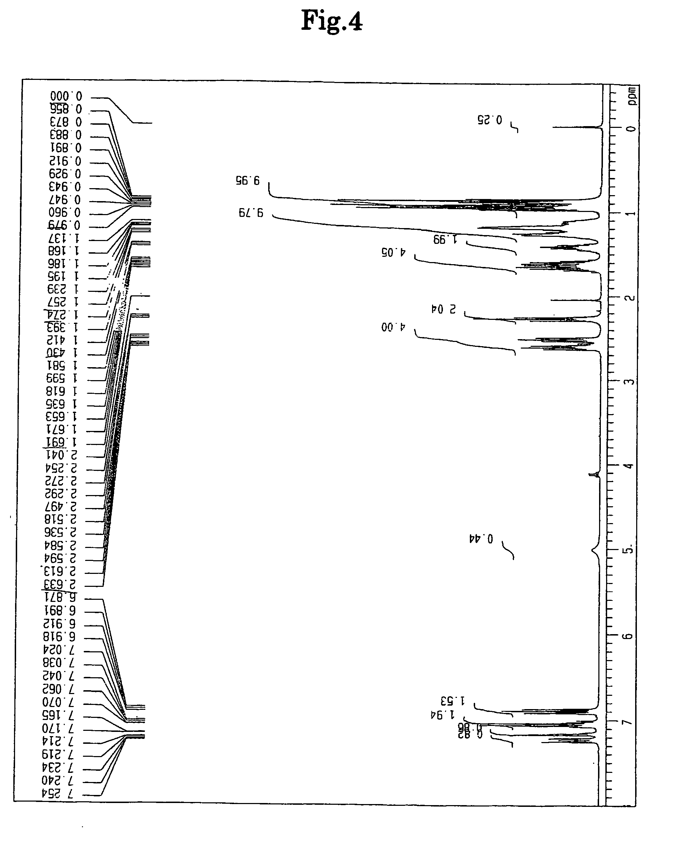

The resulting monooctanoyl derivative of propylbiphenol was identified by ¹H-NMR (400 MHz, CDCl₃) (Figure 4). The detected NMR signals are shown below.

### (Monooctanoyl derivative)

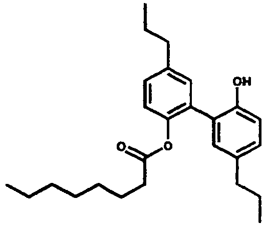

[δ (ppm) ; 0.85-0.97 (m, 9H), 1.14-1.26 (m, 8H), 1.41 (q, J = 7.2 Hz, 2H), 1.58-1.69 (m, 4H), 2.27 (t, J = 7.2 Hz, 2H), 2.49-2.63 (m, 4H), 6.87-6.91 (m, 2H), 7.02-7.07 (m, 2H), 7.23-7.25 (m, 2H)]

### Example 1 (solubility test method)

The following test was carried out to examine the solubility of the respective acyl derivatives obtained in Preparation Examples 1 to 3 in water or an organic solvent. 0.1 g of each sample was weighed and 1 ml of water or an organic solvent was accurately added thereto. After agitating the mixture with a test tube mixer (VORTEX), it was allowed to stand at room temperature and the state was visually observed. Solubility was evaluated as "o", when a sample was completely dissolved. Solubility was evaluated as "Δ", when a sample was approximately half dissolved. Solubility was evaluated as "x", when a sample was almost not dissolved. Propylbiphenol that was a starting material for each Preparation Example was also evaluated as Comparative Example 1. The results are shown in Table 1.

**[Table 1]**

| Sample compound | Character | Water | Methanol | Ethyl acetate | n-hexane |
|---|---|---|---|---|---|
| Comparative Example 1 Propylbiphenol | White powder | × | Δ | ○ | × |
| Preparation Example 1 Monoacetyl derivative | Oily compound | × | ○ | ○ | ○ |
| Preparation Example 1 Diacetyl derivative | Oily compound | × | ○ | ○ | ○ |
| Preparation Example 2 Dipropionyl derivative | Oily compound | × | ○ | ○ | ○ |
| Preparation Example 3 Monooctanoyl derivative | Oily compound | × | ○ | ○ | ○ |

It was observed from the above results that each of the acyl derivatives obtained in Preparation Examples 1 to 3 showed an improved solubility in methanol as a high-polarity solvent and hexane as a low-polarity solvent as compared with Comparative Example 1.

An IOB (inorganic-organic balance) value is a value indicating hydrophilicity of an organic compound. As the value is higher, hydrophilicity is higher. The IOB value can be calculated by the formula "IOB value = inorganic value (IV)/organic value (OV)", wherein the organic value and the inorganic value are calculated on the basis of the organic/inorganic table described in "Organic Conceptional Diagram: Fundamentals and Applications" (author: Yoshio Koda (1984), Sankyo Publishing Co., Ltd.). The OV, IV and IOB values of the respective acyl derivatives obtained in Preparation Examples 1 to 3, propylbiphenol used in Comparative Example 1 and the representative solvents are shown in Table 2.

**[Table 2]**

| Sample compound | Organic value (OV) | Inorganic value (IV) | IOB value |
|---|---|---|---|
| Comparative Example 1: Propylbiphenol | 360 | 230 | 0.64 |
| Preparation Example 1: Monoacetyl derivative | 400 | 190 | 0.48 |
| Preparation Example 1: Diacetyl derivative | 440 | 150 | 0.34 |
| Preparation Example 2: Dipropionyl derivative | 480 | 150 | 0.31 |
| Preparation Example 3: Monooctanoyl derivative | 520 | 190 | 0.37 |
| Solvent | | | |
| Methanol | 20 | 100 | 5.00 |
| Ethanol | 40 | 100 | 2.50 |
| Octanol | 160 | 100 | 0.63 |
| Acetone | 60 | 65 | 1.08 |
| Ethyl acetate | 80 | 60 | 0.75 |
| Diethyl ether | 80 | 20 | 0.25 |
| Chloroform | 140 | 30 | 0.21 |
| Benzene | 120 | 15 | 0.13 |
| Hexane | 120 | 0 | 0.00 |

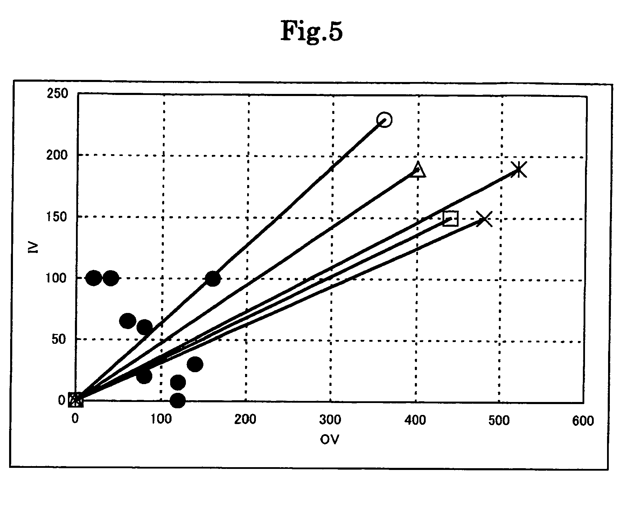

It is known that when a same proportional line is drawn passing through an IO value of a compound from the original point on an organic conceptional diagram, a solvent on the line and in a place close to the compound or a solvent not on the same line but on a proportional line in a direction close to that of the above proportional line and in a place close to the compound is a solvent in which the compound is best dissolved, generally. An organic conceptional diagram was made based on the IO values of the respective acyl derivatives obtained in Preparation Examples 1 to 3, propylbiphenol used in Comparative Example 1 and the representative solvents as shown in Table 2 (Figure 5).

Propylbiphenol of Comparative Example 1 had low solubility in methanol and n-hexane. The acyl derivatives that are the compounds of the present application had IOB values lower than that in Comparative Example 1. Accordingly, it was assumed from the conceptional diagram that the acyl derivative had increased solubility in an organic solvent and reduced solubility in methanol. However, as shown in the solubility test results, the acyl derivative had increased solubility in n-hexane and also had excellent solubility in methanol.

The following melanogenesis inhibition test was carried out using the monoacetyl and diacetyl derivatives of propylbiphenol obtained in Preparation Example 1.

### Examples 2 and 3, Comparative Examples 2 and 3 (melanogenesis inhibition test)

B16 melanoma cells were plated at 10⁴ cells/well in a MEM medium containing 10 vol% fetal bovine serum in a 12-well plate and were precultured for 24 hours. After the preculture, the medium was replaced with a medium obtained by adding a predetermined concentration of the sample compound to the above medium to which is added 2 mmol/L of theophylline, and culture was carried out for 72 hours. The cells were treated with 10 vol% trichloroacetic acid and subsequently with ethanol/diethyl ether (1:1 vol/vol). Further, the cells were dissolved in a 1 mol/L aqueous sodium hydroxide solution containing 10 vol% dimethyl sulfoxide. The OD₄₇₅ was measured and the value was used as an indicator of the amount of melanin. Thereafter, the amount of total protein in the solution was determined using Coomasie Plus Protein Assay Reagent Kit (manufactured by PIERCE) to calculate the amount of melanin per amount of protein. The melanogenesis inhibition rate (%) relative to a control was determined with a case where the sample compound was not added as the control. The test results are shown in Table 3.

**[Table 3**

| | Sample compound (Preparation Example 1) | Addition concentration µg/ml) | Melanogenesis inhibition rate (%, vs control) |
|---|---|---|---|
| Example 2 | Monoacetyl derivative | 1 | -11.6 |
| | | 3 | -9.8 |
| | | 10 | 100 |
| Example 3 | Diacetyl derivative | 1 | -15.5 |
| | | 3 | -16.6 |
| | | 10 | 72.0 |
| | | 30 | 83.8 |

As is clear from Table 3, the acylated biphenol compound of the present invention had a concentration-dependent, remarkable inhibitory effect on melanogenesis.

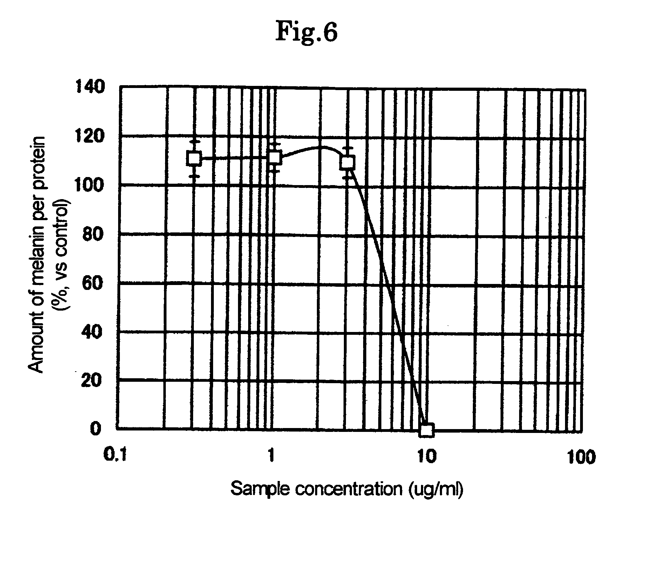

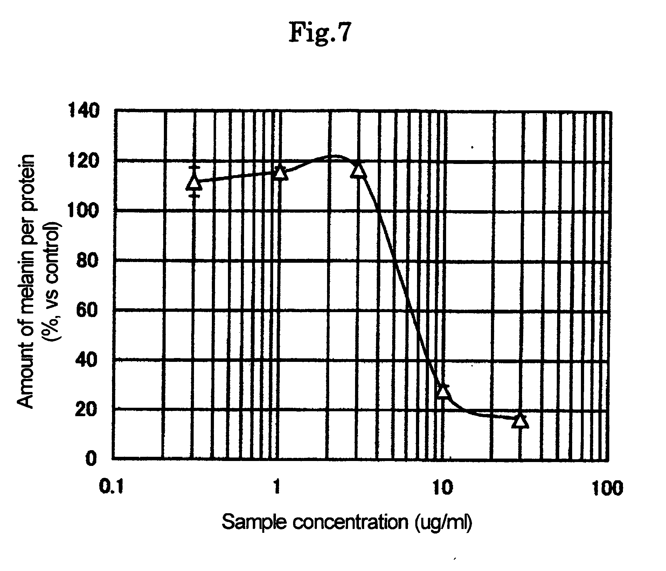

Melanogenesis inhibition curves of the monoacetyl and diacetyl derivatives of propylbiphenol of Preparation Example 1 according to the present invention were made based on the results of the melanogenesis inhibition test (Figures 6 and 7). IC₅₀ values, which is the concentration values supposed to inhibit 50% of melanogenesis, were determined from the inhibition curves. The monoacetyl derivative of Preparation Example 1 had an IC₅₀ value of 7.0 (µg/ml), and the diacetyl derivative of Preparation Example 1 had an IC₅₀ value of 6.5 (µg/ml).

Further, the above melanogenesis inhibition test was carried out using propylbiphenol which was a starting material in each Preparation Example (Comparative Example 2) and arbutin (Comparative Example 3) as samples, and the IC₅₀ values were determined by the same method. The IC₅₀ values were 4.0 (µg/ml) and 20 (µg/ml), respectively.

### Example 4, Comparative Example 4 (ultraviolet pigmentation inhibition test)

The ultraviolet pigmentation inhibitory effect of the acylated biphenol compound of the present invention was evaluated by the following ultraviolet pigmentation inhibition test using colored guinea pigs. Eight A-1 guinea pigs per one group (six weeks old at the start of the test) were used as test animals.

### 1. Ultraviolet irradiation and pigmentation evaluation method

Hair was removed from the back skin. A test site of 4 cm² (2 × 2 cm) was set and irradiated with ultraviolet rays in the UVB region. The dose per irradiation was set about 1.5 times the minimal erythema dose, and the irradiation was carried out on the first, fourth and seventh days of the test. Pigmentation was evaluated using skin luminosity (L* value) and a decrease in skin luminosity (ΔL* value) as indicators. The skin luminosity was measured using a colorimeter CR-300 (manufactured by Minolta Co., Ltd.).

### 2. Sample and experimental method

A sample containing 0.5% of the monoacetyl derivative of propylbiphenol obtained in Preparation Example 1 (Example 4) was prepared with propylene glycol/ethanol/water (20:63:17) as a base. 0.1 ml of the sample was continuously applied once a day for two weeks after the initial ultraviolet irradiation. A sample containing only the base (Comparative Example 4) was similarly applied as a control. The skin luminosity was measured before ultraviolet irradiation and two weeks after the irradiation to evaluate the effect of application of the samples on ultraviolet pigmentation in the guinea pigs.

The evaluation results are shown in Table 4. In this test system, the results indicate that ultraviolet pigmentation is so inhibited that the L* value is large and the absolute value of ΔL* is small. It was clear from the results of this test that the monoacetyl derivative of propylbiphenol of Preparation Example 1 which is the melanogenesis inhibitor of the present invention has an effect of inhibiting ultraviolet pigmentation.

**[Table 4]**

| Sample | L* value | ΔL* value | Significant difference (vs Comparative Example 1) |
|---|---|---|---|
| Example 4 | 56.59 | -7.01 | Yes (p<0.05) |
| Comparative Example 4 | 56.14 | -7.65 | - |

### (Practical whitening test method)

Skin of each flexor aspect of the forearm in each of 20 subjects was exposed to sunlight in summer season for three hours (1.5 hours per day, two days). A sample was applied to skin of flexor aspect of the left forearm and a base was applied to skin of flexor aspect of the right forearm once every morning and once every evening for 13 weeks since the day of exposure to sunlight. Evaluation was indicated as the number of subjects in which a whitening effect was confirmed in the sample application area rather than in the base application area.

### Example 5, Comparative Example 5 (skin lotion)

A skin lotion was prepared by adding an active ingredient described in Table 6 as the component (B) in the raw material composition in Table 5 to carry out the said practical whitening test.

### · Preparation method

The component B was homogeneously dissolved in the component C. Then, the components A and C were homogeneously mixed with stirring, dispersed and subsequently packed in a container.

**[Table 5]**

| Raw material component | Amount (mass%) |
|---|---|
| (A) | |
| Ethanol | 10.0 |
| Polyoxyethylene (20) sorbitan monolaurate | 5.0 |
| Dibutylhydroxytoluene | 0.01 |
| Flavor | 0.05 |

| (B) | |
|---|---|
| Component described in Table 6 or 9 | |

| (C) | |
|---|---|
| Glycerol | 5.0 |
| Xanthan gum | 0.1 |
| Hydroxyethylcellulose | 0.1 |
| Purified water | Residual amount |

**[Table 6]**

| | Component (B) | Amount (mass%) | Result of practical whitening test (number of persons) |
|---|---|---|---|
| Example 5 | Monoacetyl derivative of Preparation Example 1 | 1.0 | 15 |
| Comparative example 5 | Arbutin | 3.0 | 4 |

### · Properties

The results of the practical whitening test are described in Table 6. As shown in Table 6, an obviously good result was observed in Example 5 using the skin cosmetic product of the present invention. No subject showed skin irritation or skin sensitization.

### Example 6, Comparative Example 6 (skin cream)

A skin cream was prepared by adding an active ingredient described in Table 8 as the component (B) in the raw material composition in Table 7 to carry out the practical whitening test.

### · Preparation method

A mixture of the components A and B was homogeneously dissolved by heating so that the temperature was 80°C, respectively. Then, the component C was poured thereinto, followed by emulsification. Thereafter, the product was cooled to 30°C with stirring.

**[Table 7]**

| Raw material component | Amount (mass%) |
|---|---|
| (A) | |
| Glycerol monostearate | 2.0 |
| Beeswax | 1.0 |
| Polyoxyethylene sorbitan monooleate | 6.0 |
| Vaseline | 4.0 |
| Liquid paraffin | 12.0 |

| (B) | |
|---|---|
| Described in Table 8, 10 or 11 | |

| (C) | |
|---|---|
| Sodium N-stearoyl-L-glutamate | 1.0 |
| Carrageenan | 0.3 |
| Methylparaben | 0.3 |
| Purified water | Residual amount |

**[Table 8]**

| | Component (B) | Amount (mass%) | Result of practical whitening test (number of persons) |
|---|---|---|---|
| Example 6 | Diacetyl derivative of Preparation Example 1 | 0.5 | 17 |
| Comparative example 6 | Arbutin | 3.0 | 5 |

### · Properties

The results of the practical whitening test are described in Table 8. As shown in Table 8, an obviously good result was observed in Example 5 using the skin cosmetic product of the present invention. No subject showed skin irritation or skin sensitization.

### Example 7, Comparative Example 7 (skin lotion)

A skin lotion was prepared in the same manner as in Example 5 by adding an active ingredient described in Table 9 as the component (B) in the raw material composition in Table 5 to carry out the practical whitening test.

**[Table 9]**

| | Component (B) | Amount (mass%) | Result of practical whitening test (number of persons) |
|---|---|---|---|
| Example 7 | Monoacetyl derivative of Preparation Example 1 | 0.2 | 16 |
| | Diacetyl derivative of Preparation Example 1 | 0.2 | |
| Comparative example 7 | Vitamin C glycoside | 2.0 | 3 |

### · Properties

The results of the practical whitening test are described in Table 9. As shown in Table 9, an obviously good result was observed in Example 7 using the skin cosmetic product of the present invention. No subject showed skin irritation or skin sensitization.

### Example 8, Comparative Example 8 (skin cream)

A skin cream was prepared in the same manner as in Example 6 by adding an active ingredient described in Table 10 as the component (B) in the raw material composition in Table 7 to carry out the practical whitening test.

**[Table 10]**

| | Component (B) | Amount (mass%) | Result of practical whitening test (number of persons) |
|---|---|---|---|
| Example 8 | Diacetyl of Preparation Example 1 | 0.1 | 20 |
| | Propylbiphenol | 0.5 | |
| Comparative example 8 | Ellagic acid | 2.0 | 2 |

### · Properties

The results of the practical whitening test are described in Table 10. As shown in Table 10, an obviously good result was observed in Example 8 using the skin cosmetic product of the present invention. No subject showed skin irritation or skin sensitization.

### Example 9, Comparative Example 9 (skin cream)

A skin cream was prepared in the same manner as in Example 6 by adding an active ingredient described in Table 11 as the component (B) in the raw material composition in Table 7 to carry out the practical whitening test.

**[Table 11]**

| | Component (B) | Amount (mass%) | Result of practical whitening test (number of persons) |
|---|---|---|---|
| Example 9 | Monoacetyl derivative of Preparation Example 1 | 0.1 | 20 |
| | Diacetyl derivative of Preparation Example 1 | 0.2 | |
| | Propylbiphenol | 0.5 | |
| Comparative example 9 | Kojic acid | 2.0 | 2 |

### · Properties

The results of the practical whitening test are described in Table 11. As shown in Table 11, an obviously good result was observed in Example 9 using the skin cosmetic product of the present invention. No subject showed skin irritation or skin sensitization.

### Industrial Applicability

As described above, the melanogenesis inhibitor of the present invention has an excellent effect of inhibiting melanin pigment production, does not irritate the skin, is an oily compound, and is also excellent in stability and transdermal absorption. The present invention can provide a skin cosmetic product in various dosage forms having an excellent whitening effect and high safety.

## Claims

1. Use as a cosmetic product of an acylated biphenol compound represented by the following general formula (1): 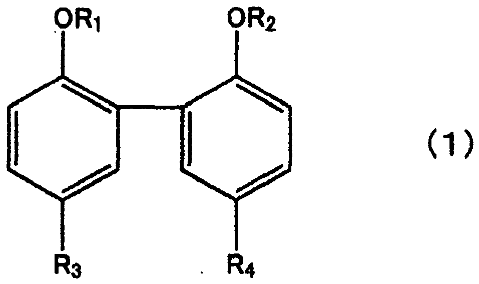 wherein
R₁ and R₂ are each a hydrogen atom or an acyl group having 2 to 10 carbon atoms, provided that both R₁ and R₂ are not hydrogen atoms; and
R₃ and R₄ are each a hydrogen atom or a linear or branched saturated hydrocarbon group having 1 to 8 carbon atoms in a method of inhibiting melanogenesis activity, wherein the product is an aqueous cosmetic product including an water-soluble organic solvent.

2. The use according to claim 1, wherein R₁ and R₂ are each a hydrogen atom or an acyl group having 2 to 8 carbon atoms, provided that both R₁ and R₂ are not hydrogen atoms; and R₃ and R₄ are each a hydrogen atom or a linear or branched saturated hydrocarbon group having 1 to 5 carbon atoms in a method of inhibiting melanogenesis activity.

3. The use according to claim 2, wherein R₁ and R₂ are each a hydrogen atom or an acyl group having 2 to 8 carbon atoms, provided that both R₁ and R₂ are not hydrogen atoms; and both R₃ and R₄ are propyl groups in a method of inhibiting melanogenesis activity.

4. A skin cosmetic product comprising a compound as defined in any one of claims 1 to 3, wherein the product is an aqueous cosmetic product including an water-soluble organic solvent, wherein the amount of the said compound is 0.001 to 10 mass %based on the total weight of the skin cosmetic product.

5. A skin cosmetic product according to claim 4, wherein the amount of the acylated biphenol compound is 0.01 to 5 mass % based on the total weight of the skin cosmetic product.

6. A skin cosmetic product according to claim 4, wherein the amount of the acylated biphenol compound is 0.01 to 8 mass %, and the amount of the water-soluble organic solvent is 1 to 40 mass %, based on the total weight of the aqueous cosmetic product.

7. A skin cosmetic product according to claim 4, wherein the water-soluble organic solvent is one or more lower alcohols selected from ethanol, propanol, isopropanol and butanol and/or one or more polyhydric alcohols selected from ethylene glycol, diethylene glycol, triethylene glycol, polyethylene glycol, propylene glycol, dipropylene glycol, polypropylene glycol, glycerol, diglycerol, polyglycerol, isoprene glycol and 1,3-butylene.

8. A skin cosmetic product according to claim 4, wherein an IOB (inorganic-organic balance) value of the water-soluble organic solvent is in the range of 1 to 7.

9. Use of an acylated biphenol compound represented by the following general formula (1): 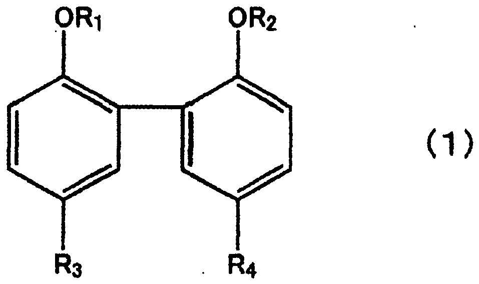 wherein
R₁ and R₂ are each a hydrogen atom or an acyl group having 2 to 10 carbon atoms, provided that both R₁ and R₂ are not hydrogen atoms; and
R₃ and R₄ are each a hydrogen atom or a linear or branched saturated hydrocarbon group having 1 to 8 carbon atoms, for the manufacture of a cosmetic composition for the inhibition of melanogenesis activity
wherein the cosmetic composition is an aqueous cosmetic composition including an water-soluble organic solvent.

## Patentansprüche

1. Verwendung einer acylierten Biphenolverbindung, die durch die folgende allgemeine Formel (1) dargestellt wird: 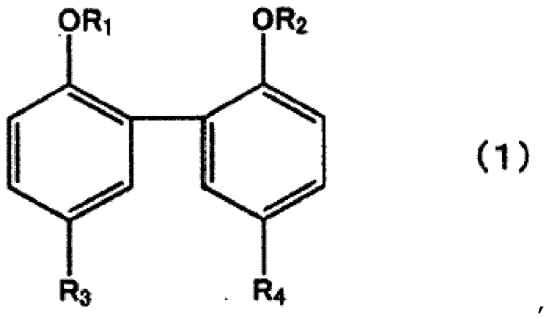 wobei
R₁ und R₂ jeweils ein Wasserstoffatom oder eine Acylgruppe mit 2 bis 10 Kohlenstoffatomen sind, vorausgesetzt, dass sowohl R₁ als auch R₂ nicht Wasserstoffatome sind; und
R₃ und R₄ jeweils ein Wasserstoffatom oder eine lineare oder verzweigte gesättigte Kohlenwasserstoffgruppe mit 1 bis 8 Kohlenstoffatomen sind,
als ein Kosmetikprodukt in einem Verfahren zum Hemmen von Melanogeneseaktivität, wobei das Produkt ein wässriges Kosmetikprodukt ist, das ein wasserlösliches organisches Lösungsmittel enthält.

2. Verwendung nach Anspruch 1, wobei R₁ und R₂ jeweils ein Wasserstoffatom oder eine Acylgruppe mit 2 bis 8 Kohlenstoffatomen sind, vorausgesetzt, dass sowohl R₁ als auch R₂ nicht Wasserstoffatome sind; und R₃ und R₄ jeweils ein Wasserstoffatom oder eine lineare oder verzweigte gesättigte Kohlenwasserstoffgruppe mit 1 bis 5 Kohlenstoffatomen sind, in einem Verfahren zum Hemmen von Melanogeneseaktivität.

3. Verwendung nach Anspruch 2, wobei R₁ und R₂ jeweils ein Wasserstoffatom oder eine Acylgruppe mit 2 bis 8 Kohlenstoffatomen sind, vorausgesetzt, dass sowohl R₁ als auch R₂ nicht Wasserstoffatome sind; und sowohl R₃ als auch R₄ Propylgruppen sind, in einem Verfahren zum Hemmen von Melanogeneseaktivität.

4. Hautkosmetikprodukt, das eine wie in einem der Ansprüche 1 bis 3 definierte Verbindung umfasst, wobei das Produkt ein wässriges Kosmetikprodukt ist, das ein wasserlösliches organisches Lösungsmittel enthält, wobei die Menge der Verbindung 0,001 bis 10 Masse-% beträgt, bezogen auf das Gesamtgewicht des Hautkosmetikprodukts.

5. Hautkosmetikprodukt nach Anspruch 4, wobei die Menge der acylierten Biphenolverbindung 0,01 bis 5 Masse-% beträgt, bezogen auf das Gesamtgewicht des Hautkosmetikprodukts.

6. Hautkosmetikprodukt nach Anspruch 4, wobei die Menge der acylierten Biphenolverbindung 0,01 bis 8 Masse-% beträgt und die Menge des wasserlöslichen organischen Lösungsmittels 1 bis 40 Masse-% beträgt, bezogen auf das Gesamtgewicht des wässrigen Kosmetikprodukts.

7. Hautkosmetikprodukt nach Anspruch 4, wobei es sich bei dem wasserlöslichen organischen Lösungsmittel um einen oder mehrere niedere Alkohole, die aus Ethanol, Propanol, Isopropanol und Butanol ausgewählt sind, und/oder einen oder mehrere mehrwertige Alkohole, die aus Ethylenglykol, Diethylenglykol, Triethylenglykol, Polyethylenglykol, Propylenglykol, Dipropylenglykol, Polypropylenglykol, Glycerin, Diglycerin, Polyglycerin, Isoprenglykol und 1,3-Butylen ausgewählt sind, handelt.

8. Hautkosmetikprodukt nach Anspruch 4, wobei ein IOB-Wert (IOB = inorganic-organic balance, Gleichgewicht anorganischer/organischer Stoffe) des wasserlöslichen organischen Lösungsmittels im Bereich von 1 bis 7 liegt.

9. Verwendung einer acylierten Biphenolverbindung, die durch die folgende allgemeine Formel (1) dargestellt wird: 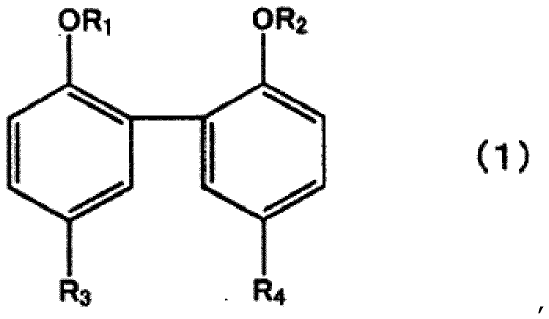 wobei
R₁ und R₂ jeweils ein Wasserstoffatom oder eine Acylgruppe mit 2 bis 10 Kohlenstoffatomen sind, vorausgesetzt, dass sowohl R₁ als auch R₂ nicht Wasserstoffatome sind; und
R₃ und R₄ jeweils ein Wasserstoffatom oder eine lineare oder verzweigte gesättigte Kohlenwasserstoffgruppe mit 1 bis 8 Kohlenstoffatomen sind,
zur Herstellung einer Kosmetikzusammensetzung zur Hemmung von Melanogeneseaktivität,
wobei die Kosmetikzusammensetzung eine wässrige Kosmetikzusammensetzung ist, die ein wasserlösliches organisches Lösungsmittel enthält.

## Revendications

1. Utilisation en tant que produit cosmétique d'un composé de bisphénol acylé illustré par la formule générale (1) suivants : 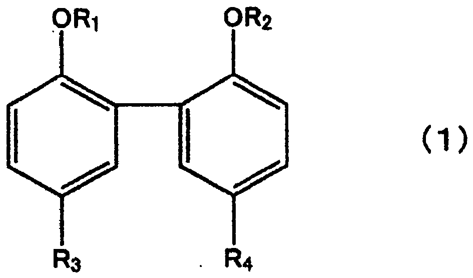 dans laquelle
R₁ et R₂ représentent chacun un atome d'hydrogène ou un groupement acyle à 2 à 10 atomes de carbone, à condition que R₁ et R₂ ne représentent pas tous deux des atomes d'hydrogène ; et
R₃ et R₄ représentent chacun un atome d'hydrogène ou un groupement hydrocarbure linéaire ou ramifié saturé à 1 à 8 atomes de carbone dans un procédé d'inhibition de l'activité de la mélanogenèse, le produit étant un produit cosmétique aqueux comprenant un solvant organique hydrosoluble.

2. Utilisation selon la revendication 1, dans laquelle R₁ et R₂ représentent chacun un atome d'hydrogène ou un groupement acyle à 2 à 8 atomes de carbone, à condition que R₁ et R₂ ne représentent pas tous deux des atomes d'hydrogène ; et R₃ et R₄ représentent chacun un atome d'hydrogène ou un groupement hydrocarbure linéaire ou ramifié saturé à 1 à 5 atomes de carbone dans un procédé d'inhibition de l'activité de la mélanogenèse.

3. Utilisation selon la revendication 2, dans laquelle R₁ et R₂ représentent chacun un atome d'hydrogène ou un groupement acyle à 2 à 8 atomes de carbone, à condition que R₁ et R₂ ne représentent pas tous deux des atomes d'hydrogène ; et R₃ et R₄ représentent des groupements propyles dans un procédé d'inhibition de l'activité de la mélanogenèse.

4. Produit cosmétique pour la peau comprenant composé selon l'une quelconque des revendications 1 à 3, le produit étant un produit cosmétique aqueux comprenant un solvant organique hydrosoluble, dans lequel la quantité du dit composé est de 0,001 à 10 % en masse sur la base du poids total du produit cosmétique pour la peau.

5. Produit cosmétique pour la peau selon la revendication 4, dans lequel la quantité de composé de bisphénol acylé est de 0,01 à 5 % en masse sur la base du poids total du produit cosmétique pour la peau.

6. Produit cosmétique pour la peau selon la revendication 4, dans lequel la quantité de composé de bisphénol acylé est de 0,01 à 8 % en masse et la quantité de solvant organique hydrosoluble de 1 à 40 % en masse sur la base du poids total du produit cosmétique aqueux.

7. Produit cosmétique pour la peau selon la revendication 4, dans lequel le solvant organique hydrosoluble est un ou plusieurs alcools inférieurs sélectionnés parmi l'éthanol, le propanol, l'isopropanol et le butanol et/ou un ou plusieurs alcools polyhydriques sélectionnés parmi l'éthylène-glycol, le diéthylène-glycol, le triéthylène-glycol, le polyéthylène-glycol, le propylène-glycol, le dipropylène-glycol, le polypropylène-glycol, le glycérol, le diglycérol, le polyglycérol, l'isoprène-glycol et le 1,3-butylène.

8. Produit cosmétique pour la peau selon la revendication 4, dans lequel une valeur d'IOB (balance inorganique-organique) du solvant organique hydrosoluble est dans la plage de 1 à 7.

9. Utilisation d'un composé de bisphénol acylé illustré par la formule générale (1) suivante : 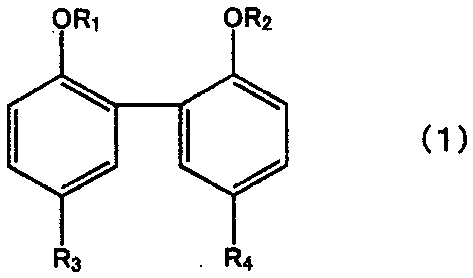 dans laquelle
R₁ et R₂ représentent chacun un atome d'hydrogène ou un groupement acyle à 2 à 10 atomes de carbone, à condition que R₁ et R₂ ne représentent pas tous deux des atomes d'hydrogène ; et
R₃ et R₄ représentent chacun un atome d'hydrogène ou un groupement hydrocarbure linéaire ou ramifié saturé à 1 à 8 atomes de carbone dans la fabrication d'une composition cosmétique pour l'inhibition de l'activité de la mélanogenèse,
la composition cosmétique étant une composition cosmétique aqueuse qui comprend un solvant organique hydrosoluble.
